# EUROPEAN PATENT SPECIFICATION

(11) **EP 0 730 020 B1**
(45) Date of publication and mention of the grant of the patent: **23.08.2000**
(21) Application number: 96301384.2
(22) Date of filing: 29.02.1996
(51) Int. Cl.: C07C 67/14, C07C 69/02, C07C 69/28, C10M 105/38, C10M 171/00, C10M 177/00

(54) **Polyol ester-based lubricant and process for the production thereof**
Schmiermittel auf Basis von Polyolester und Verfahren zu dessen Herstellung
Lubrifiant à base d'ester de polyol et son procédé de production

(30) Priority: 01.03.1995 JP 4181895; 08.12.1995 JP 32023395; 08.12.1995 JP 32023495; 08.12.1995 JP 32023595; 08.12.1995 JP 32023695
(43) Date of publication of application: 04.09.1996
(73) Proprietor: MITSUBISHI GAS CHEMICAL COMPANY, INC., Chiyoda-ku, Tokyo (JP)
(72) Inventor: Shiokawa, Yoshihiro c/o Mitsubishi Gas Chem. Co., Kurashiki-shi, Okayama-ken (JP); Matsumoto, Shunichi c/o Mitsubishi Gas Chem. Co., Kurashiki-shi, Okayama-ken (JP); Kato, Kinji c/o Mitsubishi Gas Chem. Co. Inc., Kurashiki-shi, Okayama-ken (JP)
(74) Representative: Woods, Geoffrey Corlett

(56) References cited:
- EP-A- 0 470 788
- EP-A- 0 629 603
- WO-A-90/12849
- GB-A- 2 263 481
- DATABASE WPI Section Ch, Week 9232 Derwent Publications Ltd., London, GB; Class A97, AN 92-265716 XP002023789 & JP-A-04 183 789 (SANKEN KAKO KK) , 30 June 1992
- DATABASE WPI Section Ch, Week 9638 Derwent Publications Ltd., London, GB; Class E17, AN 96-379436 XP002023790 & JP-A-08 183 973 (MITSUBISHI GAS CHEM CO INC) , 16 July 1996
- DATABASE WPI Section Ch, Week 9621 Derwent Publications Ltd., London, GB; Class E17, AN 96-205838 XP002023791 & JP-A-08 073 876 (MITSUBISHI GAS CHEM CO INC) , 19 March 1996

## Description

The present invention relates to a process for the production of a polyol ester-based lubricant suitable for use as jet engine oil, low-temperature grease base oil, heat-resistant engine oil and refrigerator oil (to be simply referred to as "refrigerator oil" hereinafter), and a process for the production thereof. In particular, it relates to polyol ester-based lubricant which has excellent compatibility with hydrofluorocarbons as a promising substitute for chlorofluorocarbons and hydrochlorofluorocarbons and has excellent hydrolysis resistance and stability against heat, and a process for the production thereof.

Chlorine-containing chlorofluorocarbons and hydrochlorofluorocarbons have been conventionally widely used as a refrigerant for refrigerators. In recent years, however, it has been found that chlorine contained in the above refrigerant destroys the ozone layer of the stratosphere, and the restriction on the use of chlorofluorocarbons and hydrochlorofluorocarbons has been being globally tightened up, and chlorine-free hydrofluorocarbons are therefore considered the most likely alternative.

On the other hand, it has been already proved that mineral oil-containing or alkylbenzene-containing refrigerator oils which have been so far used are not suitable for use with hydrofluorocarbons due to their poor compatibility with hydrofluorocarbons.

Polyol ester-based refrigerator oils excellently compatible with hydrofluorocarbons are therefore being studied for use as a substitute refrigerator oil, while the defect of ester is that it undergoes hydrolysis in the presence of water. It is therefore an urgent demand to develop an ester having excellent resistance to hydrolysis.

Polyol esters are conventionally produced by esterifying fatty acids and polyhydric alcohols under heat as is disclosed in JP-A-5-25484 and JP-A-5-70789. In this esterification, the reaction proceeds very slowly even if the reaction is carried out at a high temperature as high as 200°C or higher. It is therefore required to use a reactor having a large capacity for their industrial production.

For avoiding the reaction requiring a high temperature and a long reaction time, JP-A-4-314793, JP-A-5-27167 and JP-A-5-1291 disclose a method in which a polyol ester is produced through an acid chloride.

In the above esterification method carried out through an acid chloride, however, it is required to prepare an acid chloride from a fatty acid first, and an acid chloride such as phosphorus trichloride, phosphorus pentachloride or thionyl chloride is used. These acid chlorides are not easy to handle, and it is required to use an excess of the acid chloride relative to the fatty acid and remove the excessive acid chloride before the esterification, so that a complicated production process is required.

When used as a refrigerator oil, polyol esters obtained by the above methods have good compatibility with chlorine-free hydrofluorocarbons which are regarded as the most likely novel refrigerant, while these polyol esters are still not yet satisfactory in hydrolysis resistance which the refrigerator oil is required to have as its properties. For example, the above JP-A-5-27167 and JP-A-5-1291 disclose polyol esters in which all the α carbons of acid residue have branches as tertiary carbon atoms, and it is described that the polyol esters are excellent in stability against hydrolysis. However, as is clear from results of sealed tube tests and hydrolysis tests (JIS K2503), the above polyol esters are not necessarily satisfactory in hydrolysis resistance.

Polyol esters obtained by a method using a reaction between a fatty acid and a polyhydric alcohol or by a method carried out through an acid chloride show the property of low moisture absorption as compared with polyalkylene glycol-based refrigerator oils which are recently used in air-conditioners of automobiles. However, some refrigerator oils such as a refrigerator oil for an air conditioner for home use require the outdoor work of charging even in the time of a rainy season when the humidity is high, and such refrigerator oils require the prevention of hydrolysis of an ester for a long period of time. It is therefore required to provide a polyol ester having the property of low moisture absorption. Further, for the use with a refrigerating machine having a compressor with a motor, such as an electric refrigerating machine, the refrigerator oil is required to have not only high electrical insulation properties but also the property of low moisture absorption.

That is, when used as a refrigerator oil, polyol esters obtained by a method using a reaction between a fatty acid and a polyhydric alcohol or by a method carried out through an acid chloride have good compatibility with chlorine-free hydrofluorocarbons which are recently considered promising as a novel refrigerant, while none of the polyol esters have the property of sufficient low moisture absorption.

JP-B-7-74181 discloses a tertiary carboxylic acid composition obtained by reacting an olefin having 8 carbon atoms, carbon monoxide and water in the presence of an acid catalyst according to a Koch reaction.

It is generally known that a fatty acid in which α carbon relative to carbonyl group has branches as secondary or tertiary carbon atom, and an ester thereof, are excellent in hydrolysis resistance and stability against heat, as is disclosed in JP-A-4-314793, JP-A-5-1291, JP-A-5-17787 and JP-A-5-17789.

As described in EP-580308, etc., however, the compatibility of the above ester with hydrofluorocarbons tends to decrease with an increase in the number of branches bonding to carbon atoms in the α-position relative to carbonyl group or with an increase in the number of carbon atoms of the branches in the α-position relative to carbonyl group.

Further, as described in U. S. Patent 5,005,098, it is known that the compatibility of a polyol ester with hydrofluorocarbons increases with an increase in the number of branches of hydrocarbon chains in the fatty acid residue of the polyol ester.

The present inventors have already found that esters can be efficiently produced by reacting an olefin, carbon monoxide and an alcohol in the presence of hydrogen fluoride, and have filed a patent application therefor (EP-A-629603). This reaction for obtaining esters from an olefin, carbon monoxide and an alcohol can be carried out at a low temperature for a short period of time. Further, the hydrogen fluoride used as a catalyst can be easily separated from the reaction product by distillation and recycled, so that esters can be industrially advantageously produced.

Further, the present inventors have found that colorless and transparent esters having a decreased acid value can be obtained by catalytic hydrogenation, and have filed a patent application therefor (EP-A-629603).

In the above method, esters can be obtained as colorless and transparent ones, and the acid value of the esters can be decreased. For the use of the esters as a refrigerator oil with a substitute (hydrofluorocarbons) for hydrochlorofluorocarbons (chlorine-containing refrigerants), however, it is required to improve the esters in compatibility with the substitute, low-temperature flowability, the property of electrical insulation and the property of moisture absorption.

EP-A-470788 discloses a lubricating oil comprising esters having at least three fatty acid residues. WO 90/12849 discloses a composition containing a soluble organic lubricant containing at least one ester of the formula R[OC(O)R¹]ₙ, wherein n is at least 2, R is a hydrocarbyl group and each R¹ is hydrogen, straight chain lower hydrocarbyl, branched chain hydrocarbyl or straight chain hydrocarbyl containing from 8 to about 22 carbon atoms provided that at least one R¹ group is hydrogen, lower straight chain hydrocarbyl, branched chain hydrocarbyl or carboxylic acid- or carboxylic acid ester-containing hydrocarbyl.

GB-A-2263481 discloses a composition containing a hydrofluoroalkane refrigerant and a lubricant comprising a pentaerythritol ester. JP-A-4183789 discloses a lubricating oil involving a neo-acid ester obtained by reacting one of a variety of polyols with one of a variety of neo-acids.

As explained above, most of polyol ester-based refrigerator oils which are excellent in compatibility with a novel refrigerant, hydrofluorocarbons, are poor in hydrolysis resistance and heat stability, and most of polyol ester-based refrigerator oils which are excellent in hydrolysis resistance and heat stability are poor in compatibility with hydrofluorocarbons. It is therefore desired to urgently develop an ester which is excellent in all the properties, hydrolysis resistance, heat resistance, low-temperature flowability, electrical insulation and compatibility with hydrofluorocarbons.

It is an object of the present invention to provide a polyol ester-based lubricant as a refrigerator oil, which is excellent not only in compatibility with hydrofluorocarbons (substitute for chlorine-containing refrigerants) but also excellent in hydrolysis resistance and heat stability.

It is another object of the present invention to provide a process for the production of a neopentyl polyol ester having the above properties at high yields at a low temperature for a short period of time.

It is further another object of the present invention to provide a polyol ester-based lubricant as a refrigerator oil, which is excellent in low-temperature flowability and electrical insulation.

It is still further another object of the present invention to provide a process for the production of a neopentyl polyol ester having the above properties.

It is yet another object of the present invention to provide a polyol ester-based lubricant as a refrigerator oil, which is excellent in the property of moisture absorption at a low temperature.

Further, it is another object of the present invention to provide a process for the production of a neopentyl polyol ester having the above property.

According to the present invention, there is provided a polyol ester-based lubricant containing, as a base oil, at least one neopentyl polyol ester of the formula (I), 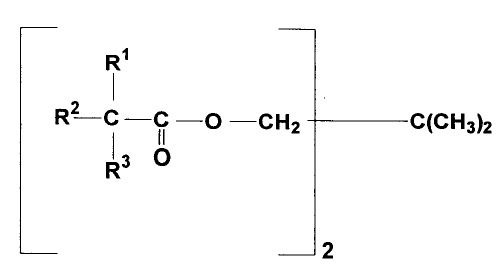 wherein R¹ is an alkyl group having at least 4 carbon atoms and at least one branch and each of R² and R³ is independently an alkyl group having 1 to 13 carbon atoms, provided that the total number of carbon atoms of R¹, R² and R³ is not more than 23, the fatty acid residues of formula (III) 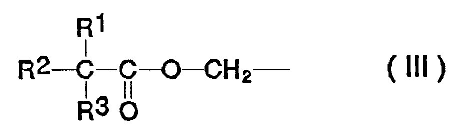 being the same or different, provided that at least 60% by weight of said at least one polyol ester contains two fatty acid residues of formula (III) containing different numbers of carbon atoms.

Also provided is a neopentyl polyol ester of formula (I), as defined above.

The present invention also provides a process for the production of a neopentyl polyol ester of the invention, which process comprises reacting neopentyl glycol with at least one fatty acid fluoride of formula (II) 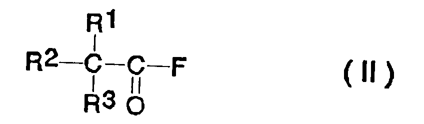 wherein R¹, R² and R³ are each as defined above,
the or each fatty acid fluoride being obtainable by reacting carbon monoxide, hydrogen fluoride and a monolefin having 4 to 12 carbon atoms wherein at least one of the carbon atoms of the olefinic double bond is a tertiary carbon atom.

Typically, said polyol ester has a pour point of -35°C or less and a volume electric resistivity of at least 1.0 x 10¹⁵ Ω.cm².

Polyol esters having such properties may be prepared by distilling a neopentyl polyol ester obtained from the process set out above, until high-boiling-point components are removed in an amount of at least 1 % by weight of the neopentyl polyol ester.

Typically, therefore, in the process of the present invention for the production of a neopentyl polyol ester, the resulting product is distilled.

Typically, said polyol ester has an acid value of 0.02 mgKOH/g or lower after 10 g of the polyol ester having an adjusted water content of 1,000 ppm or lower and an acid value of 0.02 mgKOH/g or lower is sealed in a tube with iron wire, copper wire and aluminium wire, each of which has a diameter of 0.15 mm and a length of 8 cm, and 2g of 1,1,1,2-tetrafluoroethane and allowed to stand at 200°C for 28 days.

Polyol esters having such properties may be prepared by purifying a neopentyl polyol ester obtained from the process set out above by distillation and then at 200°C for 28 days.

Polyol esters having such properties may be prepared by purifying a neopentyl polyol ester obtained from the process set out above by distillation and then hydrolysis-treating the purified neopentyl polyol ester.

Typically, therefore, in the process of the present invention for the production of a neopentyl polyol ester, the resulting product is distilled and the distilled product is further purified by hydrolysis-treatment.

In the accompanying drawing:

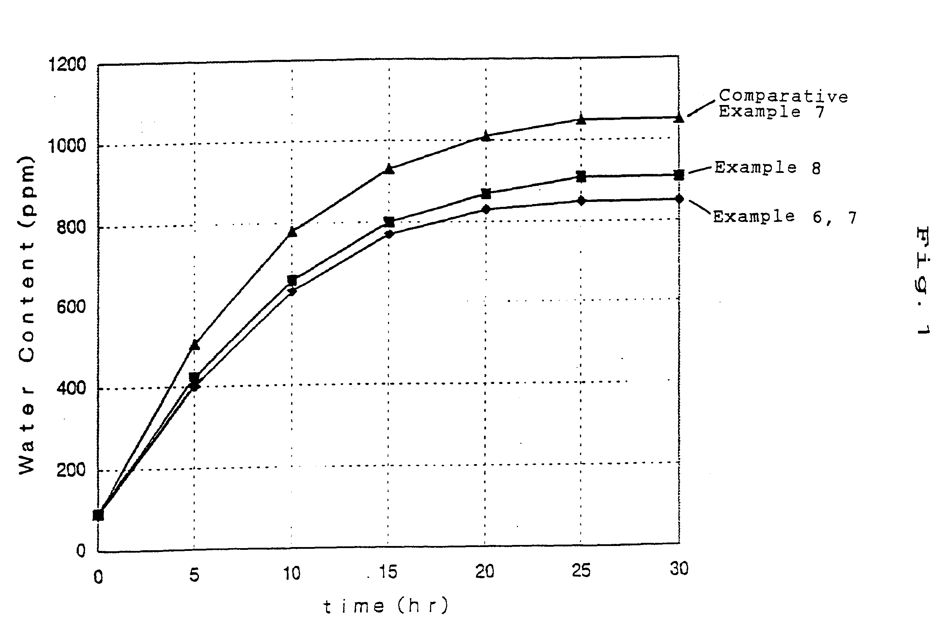

Fig. 1 is a graph showing the relationship between the measurement value of water content and the measurement time with regard to products obtained in Examples 6 ~ 8 and Comparative Example 7.

The present inventors have found the following. In polyol esters obtained from fatty acid fluorides in which a carbon atoms relative to carbonyl groups have branches as secondary or tertiary carbon atoms, a polyol ester containing fatty acid residues which are different from each other in the polyol ester molecule shows excellent compatibility with hydrofluorocarbons at a low temperature over a polyol ester containing fatty acid residues which are the same in the polyol ester molecule, and a patent application therefor has been already filed (Japanese Patent Application No. 6-327530).

The present inventors have further studied polyol ester-based lubricants, and as a result, the following has been found. An ester-based lubricant containing a mixture of neopentyl polyol esters can be obtained by reacting a monoolefin having 4 to 12 carbon atoms in which at least one carbon atom of carbon atoms constituting the double bond of the monoolefin is a tertiary carbon atom, with carbon monoxide and hydrogen fluoride to synthesize a mixture of branched fatty acid fluorides, and then reacting the mixture of branched fatty acid fluorides with neopentyl polyol. In the mixture of the neopentyl polyol esters, a polyol ester containing fatty acid residues which are different from each other and almost all of α carbon atoms relative to carbonyl groups have branches as tertiary carbon atoms. For these reasons, the above ester-based lubricant containing the mixture of the neopentyl polyol esters has excellent hydrolysis resistance and heat stability and also has compatibility with hydrofluorocarbons promising as a substitute for chlorine-containing refrigerant (chlorofluorocarbons).

The neopentyl polyol ester of the present invention has characteristic features in that it is excellent in compatibility with hydrofluorocarbons, that it shows a small saturation amount of moisture absorption, and that it is excellent in hydrolysis resistance, heat stability and flowablity at a low temperature.

The polyol ester-based lubricant of the present invention may be obtained by introducing a monoolefin having 4 to 12 carbon atoms in which at least one of carbon atoms constituting the double bond of the monoolefin is a tertiary carbon atom, into hydrogen fluoride under an elevated pressure of carbon monoxide, to synthesize a mixture of branched fatty acid fluorides (carbonylation step) and reacting the mixture of branched fatty acid fluorides with neopentyl glycol (esterification step).

In the carbonylation step, a mixture of branched fatty acid fluorides of the formula (II) set out above can be obtained.

In the above carbonylation step, a dimer of the monoolefin is actually obtained as well, and for example, when a monoolefin having 12 carbon atoms is used, the number of carbon atoms of a branched fatty acid fluoride having the largest number of carbon atoms is 25 (= 12 x 2 + 1). In addition to the formation of the dimer, a reaction also takes place in which an alkyl chain is broken, and each of R¹ to R³ is an alkyl group having 13 carbon atoms (largest). The total number of carbon atoms of R¹ to R³ is therefore 3 to 23. The isomerization of the monoolefin also takes place, and when each of R¹ to R³ is an alkyl group having at least 4 carbon atoms, the alkyl group has at least one branch. In these reactions, a mixture of various kinds of fluorides is obtained in the carbonylation step.

In the above esterification step, a mixture of neopentyl glycol diesters obtained from the above mixture of branched fatty acid fluorides and neopentyl glycol contains a large amount of, or at least 60 % by weight of, diesters each of which contains branched fatty acid residues different from each other in the number of carbon atoms. For this reason, this ester compound (mixture of the neopentyl polyol diesters) shows the compatibility with hydrofluorocarbons in a very broad temperature range as compared with an ester compound containing one kind of branched fatty acid residues.

When alkyl groups bonding to the α carbon atom relative to the carbonyl group of the branched fatty acid residue in the mixture of the polyol esters have 4 or more carbon atoms, all the alkyl groups have one or more branches. As a result, hydrocarbon chains of the branched fatty acid residue are branched to a great extent, which serves to improve the mixture of the polyol esters in the compatibility with hydrofluorocarbons.

On the other hand, the mixture of the polyol esters has excellent hydrolysis resistance and heat stability since almost all of α carbon atoms relative to carbonyl groups of branched fatty acid residues in the mixture of polyol esters have branches as tertiary carbon atoms.

In the esterification step, there is obtained a mixture of neopentyl glycol diesters in which almost all α carbon atoms relative to carbonyl groups have branches as tertiary carbon atoms, hydrocarbon chains of fatty acid residues are branched to a great extent and diesters each of which contains branched fatty acid residues different from each other in the number of carbon atoms are contained in a large amount, as large as at least 60 % by weight. That is because a mixture of branched fatty acid fluorides is synthesized form a monoolefin having 4 to 12 carbon atoms in which at least one of carbon atoms constituting the double bond of the monoolefin is a tertiary carbon atom and carbon monoxide in the presence of hydrogen fluoride in the carbonylation step. That is, when a monoolefin having 4 to 12 carbon atoms in which at least one of carbon atoms constituting the double bond of the monoolefin is a tertiary carbon atoms is reacted with carbon monoxide in a strong acid such as hydrogen fluoride, isomerization, dimerization and the cleavage of alkyl chains take place in the monoolefin, and as a result, various branched fatty acid fluorides are obtained.

An ester compound obtained by the above method has a higher viscosity than an ester compound which is obtained by reacting a fatty acid greater than the raw material olefin in the carbonylation step by one in the number of carbon atoms, with neopentyl glycol.

A polyol ester containing different fatty acid residues is generally called "hybrid" polyol ester. The mixture of polyol esters (neopentyl glycol diesters), provided by the present invention, contains a large amount, as large as at least 60 % by weight, of polyol esters containing branched fatty acid residues different from each other in the number of carbon atoms. This point is affirmed by analyzing the mixture by gas chromatography mass spectrometry (GC-MS). That is, the mixture is measured for molecular weights of individual esters, fragmentation and compositions thereof by GC-MS analysis. Fatty acid fluorides obtained in the carbonylation step are also measured for molecular weights and compositions by GS-MS analysis after methyl esterified. On the basis of these GC-MS analysis results, the polyol esters can be distinguished between diesters containing branched fatty acid residues which are the same in the number of carbon atoms and hybrid diesters containing branched fatty acid residues which are different from each other in the number of carbon atoms on the basis of molecular weights of the esters.

As described above, the mixture of polyol esters contains at least 60 % of polyol esters containing branched fatty acid residues different from each other in the number of carbon atoms, and the mixture is therefore excellent over other ester having the same viscosity as that of the mixture in all of hydrolysis resistance, heat stability and the compatibility with hydrofluorocarbons.

The monoolefin having 4 to 12 carbon atoms in which at least one of carbon atoms constituting a double bond is a tertiary carbon atom, used in the present invention, includes, for example, 2-methylpropene, 2-methyl-1-pentene, 2-methyl-2-pentene, 2-ethyl-1-hexene, 2,4,4-trimethyl-1-pentene, 2,4,4-trimethyl-2-pentene, 2-methyl-1-undecene and isomer mixtures of these olefins. Further, a commercially available product such as diisobutylene [2,4,4-trimethyl-1-pentene : 2,4,4-trimethyl-2-pentene (approximately 7 : 3) mixture] may also be used.

In the process of the present invention, the amount of the hydrogen fluoride per mole of the monoolefin can be 1 to 30 mol, preferably 3 to 15 mol, more preferably 2 to 5 mol. Thus, the or each fatty acid fluoride is typically obtainable in the presence of 2 to 5 mol of hydrogen fluoride per mole of the monoolefin. A decrease in the feed amount of the hydrogen fluoride serves to improve the volume efficiency of a reactor and decrease the heat amount and the time required for distilling and recovering the hydrogen fluoride.

In the process of the present invention, the amount of hydrogen fluoride used for the synthesis of the fatty acid fluorides per mole of the olefin can be set in the range of from 2 mol to 5 mol. In the process for the production of esters from an olefin, carbon monoxide and an alcohol in the presence of hydrogen fluoride as a catalyst, which was proposed by the present inventors in EP-A-629603, the amount of the hydrogen fluoride per mole of the olefin is required to be at least 5 mol.

In the process of the present invention, it is because of the use, as a raw material, of a monoolefin in which at least one of carbon atoms constituting the double bond of the monoolefin is a tertiary carbon atom that the amount of hydrogen fluoride can be decreased as described above. Due to the use of the above-specified olefin, the reaction rate of "inserting" carbon monoxide is increased, and a sufficient reaction rate can be attained even if the amount of hydrogen fluoride as a catalyst is decreased. Therefore, there is no economical disadvantage, nor is it observed that the amount of byproducts increases.

Further, when there is used a monoolefin in which at least one of carbon atoms constituting the double bond is a tertiary carbon atom, the reaction rate of inserting carbon monoxide is high, so that the amount of byproducts is small as compared with a case where other olefin is used. As a result, the yield of esters as an end product is high.

When the amount of the hydrogen fluoride is decreased to less than 2 mol per mole of the monoolefin as a raw material, uneconomically, the amount of byproducts is large and the reaction rate is low. The amount of the hydrogen fluoride per mole of the monoolefin may be 5 mol or more, while there is no influence or effect on the amount of byproducts, the reaction rate and the yield of fatty acid fluorides, the volume efficiency of a reactor is degraded, and a large amount of heat and a long period of time are required for recovering the hydrogen fluoride (catalyst) by distillation. The use of hydrogen fluoride in such a large amount is therefore not at all advantageous in view of economic performance.

The reaction temperature in the carbonylation step is between -50°C and 30°C, preferably between -40°C and 20°C. When the reaction temperature is too high, the amount of byproducts increases and the yield of fatty acid fluorides decreases. When the reaction temperature is too low, the reaction rate is low, and the cost for the cooling increases, which is economically disadvantageous.

The pressure of carbon monoxide in the carbonylation step is 10 to 100 kg/cm²G, preferably 10 to 80 kg/cm²G. When the pressure of carbon monoxide is low, the yield of fatty acid fluorides is low. However, when the pressure of carbon monoxide is more than 100 kg/cm²G, there is almost no further effect on the yield, and industrially disadvantageously, an additional cost is required.

The above pressure of carbon monoxide refers to a pressure of high-purity carbon monoxide. When the carbon monoxide for use contains a considerable amount of inert gas, the partial pressure of the carbon monoxide corresponds to the above pressure of carbon monoxide.

In the esterification step, the mixture of fatty acid fluorides is reacted with neopentyl glycol while the hydrogen fluoride remains, to produce neopentyl glycol ester. When the neopentyl glycol is used as an alcohol in the present invention, there is obtained a polyol ester having excellent heat stability.

The amount of the neopentyl glycol is preferably 1.0 to 1.1 mol (based on a molar amount of OH groups of the neopentyl polyol) per mole of carbon monoxide absorbed in the reaction under the above pressure. When the amount of the neopentyl glycol is too small, unreacted acid fluoride remains. When the amount of the neopentyl glycol is too large, a large amount of monoester is formed.

The reaction temperature in the above esterification step is between -50°C and 10°C, preferably between -30°C and 10°C. The esterification is carried out under elevated pressure or atmospheric pressure.

As described already, the monoolefin having 4 to 12 carbon atoms in which at least one of carbon atoms constituting the double bond is a tertiary carbon atom is used as a raw material, and this monoolefin is reacted with carbon monoxide in hydrogen fluoride, whereby a mixture of branched fatty acid fluorides of the formula (II) is obtained. Carbon atoms in α-positions relative to carbonyl groups in the above fatty acid fluorides have branches as tertiary carbon atoms, and as a result, a neopentyl polyol ester (a mixture of neopentyl polyol esters) obtained by reacting the fatty acid fluorides with a neopentyl polyol is excellent in hydrolysis resistance and heat stability, since the fatty acid residues of the neopentyl polyol ester are acid residues of a neo acid type in which carbon atoms in the α-positions have branches as tertiary carbon atoms.

Further, due to the use of the monoolefin in which at least one of carbon atoms constituting the double bond is a tertiary carbon atom, as a raw material, a reaction in which the monoolefin is dimerized and a reaction in which the alkyl chain of the monoolefin is broken take place concurrently before carbon monoxide is inserted into the monoolefin. As a result, fatty acid fluorides having various numbers of carbon atoms are obtained. Therefore, the neopentyl polyol ester obtained in the esterification step contains a large amount of neopentyl polyol esters containing branched fatty acid residues different from each other in the number of carbon atoms, and the fatty acid residues are branched to a great degree. Therefore, the neopentyl polyol ester has compatibility with hydrofluorocarbons in a broadened temperature range, particularly on a low-temperature side.

The distribution of total numbers of carbon atoms of the fatty acid fluorides can be varied by changing the hydrogen fluoride/monoolefin molar ratio, and as a result, the kinetic viscosity of the neopentyl polyol ester can be varied to a great extent.

In the distribution of total numbers of carbon atoms of the fatty acid fluorides, the amount of fatty acid fluorides having greater total numbers of carbon atoms increases with a decrease in the hydrogen fluoride/monoolefin molar ratio at the time of synthesizing the fatty acid fluorides, and the amount of fatty acid fluorides having smaller total numbers increases with an increase in the hydrogen fluoride/monoolefin molar ratio. Therefore, the smaller the hydrogen fluoride/monoolefin molar ratio at the time of synthesizing the fatty acid fluorides is, the higher kinetic viscosity of the resultant neopentyl polyol ester is. On the other hand, the greater the above hydrogen fluoride/monoolefin molar ratio is, the lower the above kinetic viscosity is. When the hydrogen fluoride/monoolefin molar ratio is 5 or more, there is almost no change in the distribution of the total numbers of carbon atoms of the fatty acid fluorides.

Further, the distribution of the total numbers of the fatty acid fluorides can be changed by changing the reaction temperature and the pressure of carbon monoxide. In this case, the lower the reaction temperature is, or the higher the pressure of carbon monoxide is, the greater the amount of the fatty acid fluorides having greater total numbers of carbon atoms is, and as a result, the resultant neopentyl polyol ester tends to have a higher kinetic viscosity.

Even when the distribution of the total numbers of carbon atoms of the fatty acid fluorides is changed for obtaining a neopentyl polyol ester having a desired kinetic viscosity by changing the hydrogen fluoride/monoolefin molar ratio, the reaction temperature and the pressure of carbon monoxide at the time of synthesizing the fatty acid fluorides, the neopentyl polyol ester always contains a large amount of neopentyl polyol esters containing branched fatty acid residues different from each other in the number of carbon atoms. Further, since each fatty acid residue of the neopentyl polyol ester is branched to a great extent, the neopentyl polyol ester has excellent compatibility with hydrofluorocarbons at a low temperature even if the kinetic viscosity is varied in a wide range.

After the esterification, hydrogen fluoride and the neopentyl polyol ester can be separated from each other by any one of a method ① in which the hydrogen fluoride is recovered by distillation and a method ② in which the reaction mixture after the esterification is poured into ice water to separate the hydrogen fluoride and the neopentyl polyol ester from each other. Industrially advantageous is the method in which the hydrogen fluoride is recovered by distillation.

The process for the ester production, provided by the present invention, can be carried out by any one of a batch method and a continuous method, while a continuous method is industrially advantageous. The conventional process for producing an ester from a fatty acid and a polyhydric alcohol can be carried out, basically, by a batch method only, and in this point, the process of the present invention is also advantageous. In the conventional process for producing an ester from a fatty acid and a polyhydric alcohol while removing formed water, the process is basically carried out by a batch method only, and when a plurality of fatty acids in which α carbon atoms differ in branching coefficient are reacted with a polyhydric alcohol, the branching coefficient causes a difference in reaction rate, so that the esterification does not proceed uniformly in some cases. In view of this point, the process for the ester production, provided by the present invention, is also advantageous.

For industrially separating hydrogen fluoride and the polyol ester from each other after the esterification, there is employed a method in which the hydrogen fluoride is recovered by distillation under the reflux of saturated hydrocarbon. The recovered hydrogen fluoride is recycled to a carbonylation reactor.

The saturated hydrocarbon is selected from hexane, heptane, octane, nonane, decane, etc. The saturated hydrocarbon works to supply heat for the decomposition of hydrogen fluoride and the polyol ester and to dilute the polyol ester in the bottom of a distillation column.

The above polyol ester from the above distillation column for recovering hydrogen fluoride is purified in a distillation column, washed with an alkali to decrease its acid value, and dehydrated by bubbling nitrogen or some other means, whereby the polyol ester purified is obtained.

Typically, the low-temperature side phase separation temperature of the said polyol ester is -70°C or lower when the polyol ester is mixed with 1,1,1,2-tetrafluoroethane(R-134a) in a 1,1,1,2-tetrafluoroethane: polyol ester weight ratio of 9:1.

Further, the said polyol ester typically has a saturation moisture absorption of 1,000 ppm or less, after standing in a closed container at 30°C and a relative humidity of 60°C for 30 hours or more. In particular, polyol esters obtainable by using neopentyl glycol in an amount of 1.0 ~ 1.1 mol (based on the molar amount of OH groups of the neopentyl glycol) per mole of carbon monoxide absorbed in the carbonylation, show a saturation moisture absorption amount of 900 ppm or lower. Preferably, therefore, the saturation moisture absorption of the polyol ester is 900 ppm or less.

In the present invention, the phase separation temperature on the low-temperature side refers to a temperature at which a mixture prepared by mixing a refrigerant (R-134a) with the polyol ester in a refrigerant/polyol ester weight ratio of 9/1 and dissolving the mixture at room temperature is phase-separated into the refrigerant and the polyol ester when gradually cooled. The phase separation temperature on the high-temperature side refers to a temperature at which the above mixture is phase separated when gradually heated.

The saturation moisture absorption amount of the polyol ester is obtained by placing the neopentyl polyol ester having an adjusted water content of 90 ppm in a glass beaker (surface area 22.9 cm²), placing the beaker in a closed container having an adjusted temperature of 30°C and an adjusted relative humidity of 60 % inside, measuring the neopentyl polyol ester for a water content at predetermined intervals of time and plotting the data.

The acid value refers to a value obtained by placing catalysts such as iron, copper and aluminum, an ester sample having an adjusted water content of 1,000 ppm or lower and an adjusted acid value of 0.02 mgKOH/g or lower and 1,1,1,2-tetrafluoroethane in a tube, closing the tube, heating the tube at 200°C for 28 days and measuring the ester sample.

In a preferred embodiment, the present invention provides a neopentyl polyol ester excellent in low-temperature flowability and electrical insulation by further distilling the reaction product of the mixture of fatty acid fluorides and a neopentyl polyol and thereby removing a predetermined amount of components having a high boiling point.

As described already, when the olefin used in the present invention is carbonylated in the presence of hydrogen fluoride, the olefin is isomerized in the hydrogen fluoride, and the polyol ester obtained therefrom is a mixture of various isomers. When hydrogen fluoride is recovered by distilling the above polyol ester under the reflux of saturated hydrocarbon, the temperature in a distillation column increases approximately up to 60 ~ 210°C in the copresence of hydrogen fluoride, depending upon the numbers of carbon atoms of the olefin as a raw material and the hydrocarbon. As a result, the polyol ester is colored yellowish. Most of components causing this coloring correspond to about 1 % by weight of components having a high boiling point. Most of these coloring components are cut by removing components having a high boiling point, whereby not only a stabilized color value is obtained, but also the low-temperature flowability is improved. Further, the polyol ester is improved in the property of electrical insulation. These components having a high boiling point include compounds having a higher boiling point than a neopentyl polyol ester having, as acid residues, two acid fluorides obtained by the dimerization of an olefin as a raw material and a trace amount of organometal compounds formed from a metal eluted from a reactor.

Accordingly, in this preferred embodiment, a mixture containing the crude polyol ester from which hydrogen fluoride has been separated and the saturated hydrocarbon can be subjected to general distillation to separate the saturated hydrocarbon and components having a low boiling point. Then at least 1 % by weight of the components having a high boiling point can be removed, and then the remaining polyol ester can be washed with an alkali or subjected to adsorption treatment for decreasing its acid value.

When at least 1 % by weight, based on the neopentyl polyol ester, of components having a high boiling point are removed by distillation, the distillation is preferably carried out at a pressure of 5 ~ 20 Torr at a bottom temperature of 210 ~ 320°C at a top temperature of 200 ~ 310°C, although these conditions depend upon the number of carbon atoms of the olefin used as a raw material.

The neopentyl polyol ester of the present invention obtainable by this preferred embodiment, has the following properties. When 1,1,1,2-tetrafluoroethane (trade name "R-134a") and the neopentyl polyol ester are mixed in a 1,1,1,2-tetrafluoroethane/neopentyl polyol ester mixing weight ratio of 9/1, the phase separation temperature of the mixture on the low-temperature side is -70°C or lower. The neopentyl polyol ester has a pour point of -35°C or lower and a volume electric resistivity of at least 1.0 x 10¹⁵ Ω.cm. The neopentyl polyol ester having these property values satisfies the properties which a lubricant base oil for hydrofluorocarbons is required to have.

The above pour point is measured according to the method specified in JIS K-2269, and the above volume electric resistivity is measured according to the method specified in JIS C-2101.

In a further preferred embodiment, the present invention provides a neopentyl polyol ester obtainable by purifying the reaction mixture of the mixture of the fatty acid fluorides and the neopentyl polyol and then hydrolysis-treating the resultant ester. The thus obtained neopentyl polyol ester has a very low acid value and has excellent hydrolysis resistance.

In this further preferred embodiment, the ester obtained by separating hydrogen fluoride can be purified by distillation and then hydrolysis-treated. When the hydrolysis treatment is carried out prior to the purification by distillation, a vessel for the hydrolysis is required to have a large capacity, which requires an additional cost. Further, components having a low boiling point, which are not required to be removed, may be decomposed to cause a side reaction.

Typically, the said hydrolysis-treatment comprises adding at least 3 % by weight, preferably to 3 to 5 % by weight, based on the reaction mixture, of water and heating to at least 190°C.

The mixture is stirred at a temperature of at least 190°C, preferably 190 to 200°C, for 3 to 5 hours to hydrolyze easily hydrolyzable ester components alone. When the amount of the above water is less than 3 % by weight, the hydrolysis rate is low so that it takes a long period of time. When the temperature is lower than 190°C, the hydrolysis treatment is insufficient and takes a very long period of time. When the temperature is higher than 200°C, the time required for the hydrolysis treatment can be decreased, while the high temperature is not industrially advantageous since the hydrolysis treatment is carried out under high pressure. The hydrolysis treatment is preferably carried out in a nitrogen-containing atmosphere for preventing oxidation.

Part of hydrolyzed esters form fatty acid carboxylic acids to increase the acid value, while the acid value can be decreased by a general method such as adsorption treatment or washing with an alkali.

For the use of the polyol ester as a lubricant base oil for hydrofluorocarbons, the polyol ester preferably has a decreased acid value when measured by a hydrolysis resistance test (sealed tube test), and it generally has an acid value of 0.02 mgKOH/g or lower. In the sealed tube test, the polyol ester of the present invention can be adjusted to an acid value of 0.02 mgKOH/g or lower, and shows remarkably excellent hydrolysis resistance.

After the esterification, a mixture of neopentyl polyol esters can be purified by a general method such as purification by distillation, adsorption treatment or hydrogenation.

The lubricant of the present invention may further comprise a conventionally used additive such as at least one of an antioxidant, a wear preventer and an epoxy compound.

According to the present invention, there is provided a polyol ester-based lubricant which makes it possible to prevent an increase in an acid value when tested for hydrolysis resistance at a high temperature for a long period of time as long as 28 days, which has the property of low moisture absorption, excellent hydrolysis resistance and heat stability and which has excellent compatibility with hydrofluorocarbons such as a refrigerant R-134a in a wide temperature range.

According to the present invention, there is provided a process for the production of a polyol ester having the above properties at high yields by a reaction of a monoolefin having 4 to 12 carbon atoms, in which at least one of carbon atoms constituting a double bond of the monoolefin is a tertiary atom, carbon monoxide, hydrogen fluoride and neopentyl polyol at a low temperature for a short period of time.

According to the present invention, further, there can be obtained an ester having a broader viscosity range than that of an ester obtained by a conventional method, from an olefin and neopentyl polyol. Thus, there is provided a polyol ester-based lubricant which has excellent compatibility with hydrofluorocarbons such as R-134a without much change when the viscosity changes, and there is provided a process for the production of the polyol ester having the above properties.

According to the present invention, furthermore, there is provided an industrially advantageous process for the production of a polyol ester, in which the hydrogen fluoride/olefin molar ratio can be decreased so that the volume efficiency of a reactor is high, and the hydrogen fluoride as a catalyst can be effectively recovered

According to a preferred embodiment of the present invention, there is provided a polyol ester-based lubricant which has low-temperature flowability and electric insulation sufficient for use as a refrigerator oil, and which has a stabilized color value, excellent hydrolysis resistance and excellent compatibility with hydrofluorocarbons, and there is provided a process for the production of the above polyol ester.

According to the above preferred embodiment, there is provided also a process for the production of neopentyl polyol ester having the above properties, in which a polyol ester is synthesized from a monoolefin having 4 to 12 carbon atoms in which at least one of carbon atoms constituting the double bond thereof is a tertiary carbon atom, carbon monoxide and neopentyl glycol in the presence of hydrogen fluoride and in which at least 1 % by weight, based on the synthesized polyol ester, of components having a high boiling point are removed by distillation.

According to a further preferred embodiment, there is provided a polyol ester-based lubricant which makes it possible to prevent an increase in an acid value when tested for hydrolysis resistance at a high temperature for a long period of time as long as 28 days, which has excellent hydrolysis resistance and which has excellent compatibility with hydrofluorocarbons, and there is provided a process for the production thereof.

According to the above further preferred embodiment, there is provided a process for the production of a neopentyl polyol ester having the above properties, in which an ester is synthesized from a monoolefin having 4 to 12 carbon atoms in which at least one of carbon atoms constituting the double bond of the monoolefin is a tertiary carbon atom, carbon monoxide and a neopentyl polyol in the presence of hydrogen fluoride, and in which the ester is rectified and then hydrolysis-treated

The process of the present invention is industrially advantageous since the reaction can be carried out at a low temperature for a short period of time and since the ester can be continuously produced through no complicated process.

The present invention will be explained more specifically with reference to Examples hereinafter, while the present invention shall not be limited to these Examples.

In Examples and Comparative Examples, esters were tested for hydrolysis resistance and compatibility with hydrofluorocarbons as follows.

### Hydrolysis resistance test:

A test tube of glass was charged with a polyol ester sample, and then, pieces of iron wire, copper wire and aluminum wire having a length of 8 cm each were placed therein. Then 10 g of a lubricant polyol ester having an adjusted water content of 1,000 ppm and 2 g of a refrigerant (R-134a, supplied by Daikin Industries, Ltd.) were placed therein, and the tube was tightly closed. The test tube was heated at 200°C for 28 days, and then the polyol ester was measured for an acid value.

Test on compatibility with refrigerant R-134a:

A test tube of glass was charged with 0.2 g of a polyol ester and 1.8 g of a refrigerant (R-134a, supplied by Daikin Industries, Ltd.), and the mixture was cooled or heated at a rate of 1°C/minute to measure a temperature at which the mixture underwent a phase separation. The cooling or heating was started at 20°C.

### Example 1

An ester was produced with a stainless steel autoclave having a stirrer, three inlet nozzles in a top portion and one outlet nozzle in a bottom and having a jacket for adjusting its internal temperature.

First, the autoclave was flushed with carbon monoxide, and then hydrogen fluoride was introduced, cooled to -20°C and pressurized up to 20 kg/cm² with carbon monoxide.

While carbon monoxide was introduced so as to maintain the reaction temperature at -20°C and the reaction pressure at 20 kg/cm², a diisobutylene mixture (2,4,4-trimethyl-1-pentene/2,4,4-trimethyl-2-pentene mixture = approximately 7/3) was introduced through a gaseous phase to synthesize a fatty acid fluoride mixture. After the completion of the introduction of the diisobutylene mixture, the mixture was continuously stirred for about 20 minutes until no absorption of carbon monoxide was observed.

The temperature in the autoclave was adjusted to -10°C and the pressure was decreased to atmospheric pressure. Then, neopentyl glycol in an equimolar amount to carbon monoxide which had been absorbed in the reaction (based on the molar amount of OH groups of the neopentyl glycol) was supplied to the autoclave and the mixture was allowed to react for 2 hours.

The reaction mixture was poured in ice water, and the mixture was washed with an alkali and then separated into an oil layer and an aqueous layer. The oil layer was purified by distillation to give a neopentyl glycol diester mixture.

The above neopentyl glycol diester mixture was analyzed with a gas chromatography mass spectrometer (GC-MS) to show that the mixture contained diesters having a molecular weight in the range of 272 ∼ 608 and that 62 % of the diesters contained fatty acid residues different from each other in the number of carbon atoms. The diester mixture was measured for a kinetic viscosity at 40°C with a Ubbellohde viscometer to show 45 cst.

### Example 2

A neopentyl glycol diester mixture was obtained by synthesizing a fatty acid fluoride mixture from 2,4,4-trimethyl-1-pentene and supplying neopentyl glycol in the same manner as in Example 1.

The above neopentyl glycol diester mixture was analyzed with a gas chromatography mass spectrometer (GC-MS) to show that the mixture contained diesters having a molecular weight in the range of 272 ∼ 608 and that 61 % of the diesters contained fatty acid residues different from each other in the number of carbon atoms. The diester mixture was measured for kinetic viscosity at 40°C with a Ubbellohde viscometer to show 43 cst.

### Comparative Example 1

A stirrer, a thermometer,a nitrogen-introducing tube, a dropping funnel and a dehydrating tube with a condenser were attached to a four-necked flask. 2,2-Dimethyloctanoic acid and 2-ethylhexanoic acid in equal amounts were charged into the flask, and converted to acid chlorides with thionyl chloride. Then, a solution of neopentyl glycol in triethylamine (the amount of OH groups of the neopentyl glycol and the amount of the acid chlorides being equimolar) was charged into the flask through the dropping funnel. The mixture was refluxed at 95°C for 4 hours, to give a neopentyl glycol diester mixture. The neopentyl glycol diester mixture was analyzed with a gas chromatography mass spectrometer (GC-MS) to show that the mixture contained diesters having a molecular weight in the range of 356 ∼ 412 and that 49 % of the diesters contained fatty acid residues different from each other in the number of carbon atoms. The diester mixture was measured for a kinetic viscosity at 40°C with a Ubbellohde viscometer to show 15 cst.

### Comparative Example 2

An equimolar mixture of 2,2-dimethyloctanoic acid and 2,2-dimethylpentanoic acid was converted to acid chlorides, and allowed to esterification-react with neopentyl glycol, in the same manner as in Comparative Example 1 to give a neopentyl glycol diester mixture. The neopentyl glycol diester mixture was analyzed with a gas chromatography mass spectrometer (GC-MS) to show that the mixture contained diesters having a molecular weight in the range of 328 ∼ 412 and that 46 % of the diesters contained fatty acid residues different from each other in the number of carbon atoms. The diester mixture was measured for a kinetic viscosity at 40°C with a Ubbellohde viscometer to show 16 cst.

### Comparative Example 3

In the same manner as in Comparative Example 1,3,5,5-trimethylhexanoic acid and trimethylolpropane were esterified to give trimethylolpropane-3,5,5-trimethylhexanoate. The ester was measured for a kinetic viscosity at 40°C with a Ubbellohde viscometer to show 52 cst.

Table 1 shows the results of the hydrolysis resistance test of the polyol esters (neopentyl glycol diester mixtures) obtained in the above Examples 1 ~ 2 and Comparative Examples 1 ∼ 3. Table 2 shows the results of the test of the compatibility with R-134a (1,1,1,2-tetrafluoroethane). In Table 1, values in "Acid value before test" shows acid values of the esters immediately after the esters were produced, and values in "Acid value after test" show acid values of the esters after the esters were heated for 28 days.

In the hydrolysis resistance test, the esters were heated at 200°C for a long period of time, and the results of the hydrolysis resistance test therefore show heat stability as well.

The esters obtained in Comparative Examples are generally poor in hydrolysis resistance as compared with the esters obtained in Examples, and it is seen that the ester obtained in Comparative Example 2 which is relatively superior in hydrolysis resistance is poor in compatibility with R-134a on a low-temperature side.

**Table 1**

| | Acid value before hydrolysis resistance test mgKOH/g | Acid value after hydrolysis resistance test mgKOH/g |
|---|---|---|
| Ex. 1 | 0.01 | 0.35 |
| Ex. 2 | 0.01 | 0.33 |
| CEx. 1 | 0.01 | 1.4 |
| CEx. 2 | 0.01 | 0.83 |
| CEx. 3 | 0.01 | 2.8 |
| Ex. = Example, CEx. = Comparative Example | | |

**Table 2**

| | Phase separation temperature on low-temperature side °C | Phase separation temperature on high-temperature side °C | Kinetic viscosity (40°C) cst |
|---|---|---|---|
| Ex. 1 | <-70 | >70 | 45 |
| Ex. 2 | <-70 | >70 | 43 |
| CEx. 1 | -60 | >70 | 15 |
| CEx. 2 | -40 | >70 | 16 |
| CEx. 3 | -52 | >70 | 52 |
| Ex. = Example, CEx. = Comparative Example | | | |

### Example 3

An ester was produced with a stainless steel autoclave having a volume of 10 liters and having a stirrer, three inlet nozzles in a top portion and one outlet nozzle in a bottom, having a jacket for adjusting its internal temperature.

First, the autoclave was flushed with carbon monoxide, and then 2,100 g (105.0 mo) of hydrogen fluoride was introduced, cooled to -30°C and pressurized up to 20 kg/cm² with carbon monoxide.

While carbon monoxide was introduced so as to maintain the reaction temperature at -30°C and the reaction pressure at 20 kg/cm², a diisobutylene mixture (2,4,4-trimethyl-1-pentene/2,4,4-trimethyl-2-pentene mixture = approximately 7/3) was introduced through a gaseous phase to synthesize a fatty acid fluoride mixture. After the completion of the introduction of the diisobutylene, the mixture was continuously stirred for about 20 minutes until no absorption of carbon monoxide was observed. The amount of the absorbed carbon monoxide was 600 g (21.4 mol) on the basis of a weight difference in a carbon monoxide measuring tank.

Then, the pressure was decreased to atmospheric pressure to purge unreacted carbon monoxide. Then, 1,113 g (10.7 mol) of neopentyl glycol was supplied to the autoclave over about 20 minutes, and the mixture was stirred for 1 hour. After the completion of the reaction, the reaction mixture was supplied to a hydrogen fluoride recovery distillation column, where hydrogen fluoride was recovered through a column top and a crude neopentyl polyol ester was recovered through a column bottom. The obtained crude neopentyl polyol ester was rectified in a rectification column to give 4,244 g of purified neopentyl polyol ester. The yield thereof based on the supplied diisobutylene mixture, carbon monoxide and neopentyl glycol was 98 %.

The above neopentyl polyol ester was analyzed with a gas chromatography mass spectrometer (GC-MS) to show that it was a mixture containing neopentyl glycol diesters having a molecular weight in the range of 272 ∼ 608 and that it had a purity of 99 % or higher. The diester mixture was measured for a kinetic viscosity at 40°C with a Ubbellohde viscometer to show 58 cst.

### Example 4

Fatty acid fluorides were synthesized in the same manner as in Example 3 except that the amount of the diisobutylene mixture was changed to 3,920 g (35.9 mol). After the completion of the supply of the diisobutylene mixture, the reaction mixture was continuously stirred for about 20 minutes until no absorption of carbon monoxide was found. The amount of the absorbed carbon monoxide was 862 g (30.8 mol) on the basis of a weight difference in a carbon monoxide measuring tank.

Then, 1,602 g (15.4 mol) of neopentyl glycol was supplied, the reaction was carried out, and the reaction mixture was treated, in the same manner as in Example 3, to give 6,192 g of a purified neopentyl polyol ester. The yield thereof based on the supplied diisobutylene mixture, carbon monoxide and neopentyl glycol was 97 %.

The above neopentyl polyol ester was analyzed with a gas chromatography mass spectrometer (GC-MS) to show that it was a mixture containing neopentyl glycol diesters having a molecular weight in the range of of 272 ∼ 608 and that it had a purity of 99 % or higher. The diester mixture was measured for a kinetic viscosity at 40°C with a Ubbellohde viscometer to show 94 cst.

### Example 5

Fatty acid fluorides were synthesized in the same manner as in Example 3 except that the temperature at the time of synthesizing the fatty acid fluorides was changed to 0°C. After the completion of the supply of the diisobutylene mixture, the reaction mixture was continuously stirred for about 20 minutes until no absorption of carbon monoxide was found. The amount of the absorbed carbon monoxide was 683 g (24.4 mol) on the basis of a weight difference in a carbon monoxide measuring tank.

Then, 1,269 g (12.2 mol) of neopentyl glycol was supplied, the reaction was carried out, and the reaction mixture was treated, in the same manner as in Example 3, to give 4,478 g of a purified neopentyl polyol ester. The yield thereof based on the supplied diisobutylene mixture, carbon monoxide and neopentyl glycol was 98 %.

The above neopentyl polyol ester was analyzed with a gas chromatography mass spectrometer (GC-MS) to show that it was a mixture containing neopentyl glycol diesters having a molecular weight in the range of 272 ∼ 608 and that it had a purity of 99 % or higher. The diester mixture was measured for a kinetic viscosity at 40°C with a Ubbellohde viscometer to show 15 cst.

### Comparative Example 4

Fatty acid fluorides were synthesized in the same manner as in Example 3 except that the diisobutylene mixture was replaced with 907 g (8.1 mol) of 1-octene and that the temperature at the time of synthesizing a mixture of the fatty acid fluorides was changed to -18°C. The amount of the absorbed carbon monoxide was 176 g (6.3 mol) on the basis of a weight difference in a carbon monoxide measuring tank.

Then, 328 g (3.2 mol) of neopentyl glycol was supplied, the reaction was carried out, and the reaction mixture was treated, in the same manner as in Example 3, to give 1,149 g of a purified neopentyl polyol ester. The yield thereof based on the supplied 1-octene, carbon monoxide and neopentyl glycol was 81 %.

The above neopentyl polyol ester was analyzed with a gas chromatography mass spectrometer (GC-MS) to show that it was an isomer mixture of neopentyl glycol diesters having a molecular weight of 384 and that it had a purity of 99 % or higher. The diester mixture was measured for a kinetic viscosity at 40°C with a Ubbellohde viscometer to show 10 cst.

### Comparative Example 5

A 1-liter four-necked flask was equipped with a stirrer, a thermometer, a nitrogen introducing tube and a dehydration tube with a cooling tube. The flask was charged with 104 g (1.0 mol) of neopentyl glycol, 348 g (2.2 mol) of 2,2-dibutylheptanoic acid and a catalytic amount of p-toluenesulfonic acid. The mixture was allowed to react in an esterification reaction under nitrogen current at 240°C for 12 hours while removing formed water. After the completion of the reaction, the resultant reaction mixture was extracted with n-hexane, the extract was washed with a 5 % potassium hydroxide aqueous solution and further washed with water, and the solvent was distilled off to give 346 g of neopentyl glycol ester.

The above neopentyl glycol ester was analyzed with a gas chromatography mass spectrometer (GC-MS) to show that it was a neopentyl glycol di-2,2-dimethyl heptanoate having a molecular weight of 384. The ester was measured for a kinetic viscosity at 40°C with a Ubbellohde viscometer to show 27 cst. The yield thereof based on the neopentyl glycol was 90 %.

### Comparative Example 6

A 1-liter four-necked flask having a stirrer, a thermometer, a dropping funnel and a Dimroth condenser was charged with 160 ml of toluene and 324 g (2.4 mol) of thionyl chloride, and the mixture was maintained at 50°C under nitrogen current. While the mixture was stirred, a mixture containing 316 g (2 mol) of 2,2-dimethylheptanoic acid and 40 ml of toluene was dropwise added over 1 hour, to generate heat and to generate SO₂ gas and HCl gas. After the completion of the addition, no generation of gases was found in 1 hour, and then, the Dimroth condenser was replaced with a distillation head, and the reaction mixture was temperature-increased to 95°C to distill off excessive thionyl chloride. Again, the distillation head was replaced with the Dimroth condenser, and then, a mixture containing 104 g (1.0 mol) of neopentyl glycol and 200 ml of triethylamine was dropwise added to generate heat. After the completion of the addition, the mixture was refluxed for 4 hours, and then cooled to room temperature to form a precipitate. The precipitate was removed by filtration, and then the remaining reaction mixture was washed with a 5 % potassium hydroxide aqueous solution and further was washed with water. Then, the solvent was distilled off to give 357 g of neopentyl glycol ester.

The above neopentyl glycol ester was analyzed with a gas chromatography mass spectrometer (GC-MS) to show that it was a neopentyl glycol di-2,2-dimethyl heptanoate having a molecular weight of 384. The ester was measured for a kinetic viscosity at 40°C with a Ubbellohde viscometer to show 27 cst. The yield thereof based on the neopentyl glycol was 93 %.

**Table 3**

| | Acid value before hydrolysis resistance test mgKOH/g | Acid value after hydrolysis resistance test mgKOH/g |
|---|---|---|
| Ex. 3 | 0.01 | 0.30 |
| Ex. 4 | 0.01 | 0.29 |
| Ex. 5 | 0.01 | 0.28 |
| CEx. 4 | 0.01 | 1.90 |
| CEx. 5 | 0.01 | 0.82 |
| CEx. 6 | 0.01 | 0.81 |
| Ex. = Example, CEx. = Comparative Example | | |

**Table 4**

| | Phase separation temperature on low-temperature side °C | Phase separation temperature on high-temperature side °C |
|---|---|---|
| Ex. 3 | -72 | 70< |
| Ex. 4 | -71 | 70< |
| Ex. 5 | -77 | 70< |
| CEx. 4 | -40 | 70< |
| CEx. 5 | -50 | 70< |
| CEx. 6 | -50 | 79< |
| Ex. = Example, CEx. = Comparative Example | | |

As shown in Comparative Example 4, when a linear olefin is used as a raw material (olefin), the yield of an ester as an end product is low, and the ester is poor in hydrolysis resistance and the compatibility with R-134a. Further, it is difficult to decrease the hydrogen fluoride/olefin molar ratio.

As shown in Comparative Example 5, when an ester is produced directly from neo-acid and neopentyl glycol by general esterification, it is required to carry out the reaction at a high tempreature of at least 200°C for more than 10 hours. The volume efficiency of the reactor is poor, and the ester is poor in the compatibility with R-134a.

Further, as shown in Comparative Example 6, the process carried out through an acid chloride requires complicated procedures in which excessive thionyl chloride is removed before the esterification and a solid is removed by filtration after the esterification. The ester is poor in the compatibility with R-134a.

### Measurement of saturation absorption amount:

The water content of a polyol ester sample was adjusted by nitrogen bubbling such that the sample had an initial water concentration of 90 ppm, and the sample was placed in a glass beaker (surface area 22.9 cm²). The beaker was placed in a closed container which was adjusted to a temperature of 30°C and also adjusted to a relative humidity of 60 % with a humidifier, and the polyol ester sample was measured for a water content at predetermined intervals of time. The obtained data were plotted to determine a saturated absorption amount.

### Example 6

A mixture of acid fluorides was synthesized in the same manner as in Example 3 except that the amount of hydrogen fluoride was changed from 105 mol (2,100 g) to 100 mol (2,000 g) and that the amount of the disobutylene mixture (2,4,4-trimethyl-1-pentene/2,4,4-trimethyl-2-pentene mixture = approximately 7/3) was changed from 23.3 mol (2,618 g) to 28.6 mol (3,203 g). In this case, the amount of absorbed carbon monoxide was 26.3 mol.

The temperature inside the autoclave was adjusted to -10°C, and the pressure in the autoclave was decreased to atmospheric pressure. Neopentyl glycol in an equimolar amount (based on the molar amount of OH groups of neopentyl glycol) to the carbon monoxide absorbed in the reaction was supplied to the autoclave, and the mixture was allowed to react for 2 hours.

The reaction mixture was supplied to a hydrogen fluoride recovery distillation column, where hydrogen fluoride was recovered through a column top and a crude neopentyl polyol ester was recovered through a column bottom. The crude neopentyl polyol ester was rectified with a distillation column, washed with an alkali to decrease its acid value, and dehydrated by nitrogen bubbling to give a purified neopentyl polyol ester.

The above-obtained neopentyl polyol ester had an acid value of 0.01 mg/KOH/g and a water content of 90 ppm. Further, the ester was measured for a kinetic viscosity at 40°C with a Ubbellohde viscometer to show 72 cst.

### Example 7

A neopentyl polyol ester was obtained in the same manner as in Example 6 except that the amount of carbon monoxide was changed to 1.1 mol per mole of the absorbed carbon monoxide. The ester was measured for a kinetic viscosity at 40°C with a Ubbellohde viscometer to show 70 cst.

### Example 8

A neopentyl polyol ester was obtained in the same manner as in Example 6 except that the amount of carbon monoxide was changed to 1.3 mol per mole of the absorbed carbon monoxide. The ester was measured for a kinetic viscosity at 40°C with a Ubbellohde viscometer to show 68 cst.

### Comparative Example 7

A 1-liter four-necked flask was equipped with a stirrer, a thermometer, a nitrogen introducing tube and a dehydration tube with a condenser. The flask was charged with 0.5 mol (68 g) of pentaerythritol and 2.0 mol (288 g) of 2-ethylhexanoic acid, and the mixture was allowed to react for esterification under nitrogen current at 240°C for 10 hours, to give a tetraester. The ester was measured for a kinetic viscosity at 40°C with a Ubbellohde viscometer to show 45 cst.

Similarly, the same flask as the above was charged with 0.5 mol (68 g) of pentaerythritol and 2.0 mol (316 g) of 3,5,5-trimethylhexanoic acid, and the mixture was allowed to react in the same manner as above, to give a tetraester. The ester was measured for a kinetic viscosity at 40°C with a Ubbellohde viscometer to show 114 cst.

The above tetraesters were mixed such that the mixture had a kinetic viscosity of 68 cst at 40°C, and the mixture was further adjusted to an acid value of 0.01 mgKOH/g and a water content of 90 ppm in the same manner as in Example 6.

**Table 5**

| | Acid value before hydrolysis resistance test mgKOH/g | Acid value after hydrolysis resistance test mgKOH/g |
|---|---|---|
| Ex. 6 | 0.01 | 0.30 |
| Ex. 7 | 0.01 | 0.31 |
| Ex. 8 | 0.01 | 0.40 |
| CEx. 7 | 0.01 | 3.00 |
| Ex. = Example, CEx. = Comparative Example | | |

**Table 6**

| | Phase separation temperature on low-temperature side °C | Phase separation temperature on high-temperature side °C | Kinetic viscosity (40°C) cst |
|---|---|---|---|
| Ex. 6 | -71 | 70< | 72 |
| Ex. 7 | -73 | 70< | 70 |
| Ex. 8 | -73 | 70< | 68 |
| CEx. 7 | -33 | 70< | 68 |
| Ex. = Example, CEx. = Comparative Example | | | |

**Table 7**

| Measured water content (ppm) | | | | | | | | |
|---|---|---|---|---|---|---|---|---|
| Hour | 0 | 5 | 10 | 15 | 20 | 25 | 30 | *1 |
| Ex. 6 | 90 | 398 | 630 | 770 | 831 | 850 | 850 | 850 |
| Ex. 7 | 90 | 400 | 631 | 772 | 831 | 850 | 850 | 850 |
| Ex. 8 | 90 | 421 | 657 | 800 | 870 | 910 | 910 | 910 |
| CEx. 7 | 90 | 503 | 780 | 932 | 1,010 | 1,050 | 1,050 | 1,050 |
| Ex. = Example, CEx. = Comparative Example | | | | | | | | |

| | | | | | | | | |
|---|---|---|---|---|---|---|---|---|
| *1 = Saturation moisture absorption amount | | | | | | | | |

Fig. 1 shows relationships (plotted) between measured water contents and measurement hours of time.

The neopentyl polyol ester mixtures obtained in above Examples and Comparative Examples were measured for pour points according JIS K-2269, for color values according to a Saybolt color testing method (JIS K2508), and for volume resistivity at 25°C (for electric insulation properties) according to JIS-C2101. The greater the volume electric resistivity is, the superior the property of electrical insulation is.

### Example 9

A mixture of acid fluorides was synthesized in the same manner as in Example 6 except that the amount of a diisobutylene mixture (2,4,4-trimethyl-1-pentene/2,4,4-trimethyl-2-pentene mixture = approximately 7/3) was changed from 28.6 mol (3,203 g) to 27 mol (3,024 g). In this case, the amount of absorbed carbon monoxide was 24.8 mol. Then, the mixture of acid fluorides and neopentyl glycol were allowed to react in the same manner as in Example 6.

The reaction mixture was supplied to a hydrogen fluoride recovery distillation column and distilled under the reflux of n-decane. Hydrogen fluoride was recovered through a column top, and a crude neopentyl polyol ester and n-decane were recovered through a column bottom. The obtained column bottom solution was distilled to cut the n-decane and components having a low boiling point.

Then, the neopentyl polyol ester after the above removal of components having a low boiling point was simple-distilled to remove 1 % by weight thereof as a still residue. The distillate was washed with an alkali to decrease the acid value of the neopentyl polyol ester to 0.01 mgKOH/g. Further, the neopentyl polyol ester was adjusted to a water content of 200 ppm by bubbling nitrogen. The neopentyl polyol ester was measured for a kinetic viscosity at 40°C with a Ubbellohde viscometer to show 68 cst.

### Example 10

A neopentyl polyol ester was obtained in the same manner as in Example 9 except that 22 mol (2,464 g) of 2,4,4-trimethyl-1-pentene alone was supplied as a raw material. The neopentyl polyol ester was measured for a kinetic viscosity at 40°C with a Ubbellohde viscometer to show 58 cst.

### Example 11

Neopentyl polyol ester was obtained in the same manner as in Example 9 except that the removal of components having a high boiling point was not carried out. The neopentyl polyol ester was measured for a kinetic viscosity at 40°C with a Ubbellohde viscometer to show 70 cst.

### Example 12

Neopentyl polyol ester was obtained in the same manner as in Example 9 except that the removal of components having a high boiling point was not carried out. To this neopentyl polyol ester was added 3 % by weight of a 5 % Ru/C powder, and the neopentyl polyol ester was catalytically hydrogenated at a hydrogen pressure of 8 kg/cm² G at a temperature of 80°C for 2 hours. The hydrogenated neopentyl polyol ester was measured for a kinetic viscosity at 40°C with a Ubbellohde viscometer to show 71 cst.

**Table 8**

| | Pour point (°C) | Volume resistivity (Ω·cm) | Saybolt Color |
|---|---|---|---|
| Ex. 9 | -37.5 | 1.3 x 10¹⁵ | +28 |
| Ex. 10 | -37.5 | 1.1 x 10¹⁵ | +28 |
| Ex. 11 | -27.5 | 2.3 x 10¹³ | +15 |
| Ex. 12 | -32.5 | 7.0 x 10¹³ | +28 |
| Ex. = Example, CEx. = Comparative Example | | | |

**Table 9**

| | Acid value before hydrolysis resistance test mgKOH/g | Acid value after hydrolysis resistance test mgKOH/g |
|---|---|---|
| Ex. 9 | 0.01 | 0.30 |
| Ex. 10 | 0.01 | 0.31 |
| Ex. 11 | 0.01 | 0.31 |
| Ex. 12 | 0.01 | 0.32 |
| Ex. = Example, CEx. = Comparative Example | | |

**Table 10**

| | Phase separation temperature on low-temperature side °C | Phase separation temperature on high-temperature side °C | Kinetic viscosity (40°C) cst |
|---|---|---|---|
| Ex. 9 | -71 | 70< | 68 |
| Ex. 10 | -72 | 70< | 58 |
| Ex. 11 | -71 | 70< | 70 |
| Ex. 12 | -71 | 70< | 71 |
| Ex. = Example, CEx. = Comparative Example | | | |

### Example 13

A mixture of acid fluorides was synthesized in the same manner as in Example 6 except that the amount of the diisobutylene mixture (2,4,4-trimethyl-1-pentene/2,4,4-trimethyl-2-pentene mixture = approximately 7/3) was changed from 28.6 mol (3,203 g) to 25 mol (2,800 g). In this case, the amount of absorbed carbon monoxide was 23.0 mol. Then, the mixture of acid fluorides and neopentyl glycol were allowed to react in the same manner as in Example 6 to obtain a crude neopentyl polyol ester, and the crude neopentyl polyol ester was rectified with a rectification column.

For hydrolysis, water in an amount of 5 % by weight of based on the purified neopentyl polyol ester was charged into a stirring vessel together with the neopentyl polyol ester, and the reactor was flushed with nitrogen. Then, the mixture was stirred at 200°C for 3 hours. After cooled, the reaction mixture was withdrawn, and washed with an alkali to decrease the acid value of the neopentyl polyol ester to 0.01 mgKOH/g. The water content of the neopentyl polyol ester was adjusted to 200 ppm by bubbling nitrogen. The neopentyl polyol ester was measured for a kinetic viscosity at 40°C with a Ubbellohde viscometer to show 63 cst.

### Example 14

A neopentyl polyol ester was obtained in the same manner as in Example 13 except that the amount of water for hydrolysis was changed to 3.5 % by weight, that the temperature for the hydrolysis was changed to 195°C and that the time for the hydrolysis was changed to 5 hours. The neopentyl polyol ester was measured for a kinetic viscosity at 40°C with a Ubbellohde viscometer to show 62 cst.

### Example 15

A neopentyl polyol ester was obtained in the same manner as in Example 13 using a diisobutylene mixture except that no hydrolysis was carried out. The neopentyl polyol ester was measured for a kinetic viscosity at 40°C with a Ubbellohde viscometer to show 65 cst.

### Examples 16 and 17

Neopentyl polyol esters were obtained in the same manner as in Example 13 except that the temperature of hydrolysis was decreased to 180°C and that the time for the hydrolysis was changed as shown in Table 11. Each neopentyl polyol esters was measured for a kinetic viscosity at 40°C with a Ubbellohde viscometer to show 64 cst.

### Examples 18 and 19

Neopentyl polyol esters were obtained in the same manner as in Example 13 except that the amount of water for the hydrolysis was changed to 2 % by weight based on purified neopentyl polyol esters and the time for the hydrolysis was changed as shown in Table 11. Each neopentyl polyol ester was measured for a kinetic viscosity at 40°C with a Ubbellohde viscometer to show 63 cst.

### Example 20

The same autoclave as that used in Example 13 was charged with 27 g of a mixture solution containing 64 % by weight of sulfuric acid, 29 % by weight of phosphoric acid and 7 % by weight of water and 2.3 g of cupric oxide, and the autoclave was flushed with carbon monoxide. Then, the mixture was stirred at a rate of 1,000 rpm for 3 hours at a temperature of 25°C while maintaining the pressure at 20 kg/cm² with carbon monoxide, to dissolve the cupric oxide.

Then, while the above temperature and the above pressure were maintained, 33.6 g of a C₈ olefin mixture (mixture of 70.9 % by weight of 2,4,4-trimethyl-1-pentene, 22.1 % by weight of 2,4,4-trimethyl-2-pentene and 7.0 % by weight of other C₈ olefin) was supplied into the autoclave with a pump over 90 minutes. Carbon monoxide absorbed as the reaction proceeded was compensated by maintaining a pressure regulating valve at 15 kg/cm² G.

After the supply of the diisobutylene mixture was terminated, the reaction mixture was further stirred for 1 hour.

After the completion of the reaction, residual carbon monoxide in the autoclave was removed, and the formed reaction mixture was diluted with water in an amount 3 times as large as the reaction mixture. The diluted reaction mixture was extracted with 200 ml of n-hexane three times. The n-hexane was removed from the extract, to give a crude carboxylic acid mixture.

A polyol ester was synthesized from the above crude carboxylic acid mixture and neopentyl glycol as follows.

A 500-ml four-necked flask having a stirrer, a thermometer, a dropping funnel and a Dimroth condenser was charged with 80 ml of toluene and 12.93 g of thionyl chloride, and the mixture was maintained at 55°C under nitrogen current. While the mixture was stirred, 21.65 g of the above crude carboxylic acid mixture and 20 ml of toluene were dropwise added gradually, to generate heat and to generate SO₂ gas and HCl gas.

After the completion of the addition, no generation of gases was found in about 1 hour, and then, the Dimroth condenser was replaced with a distillation head, and the reaction mixture was temperature-increased to 95°C to distill off excessive thionyl chloride. Again, the distillation head was replaced with the Dimroth condenser, and then, a mixture containing 36.85 g of neopentyl glycol and 200 ml of triethylamine was dropwise added to generate heat. After the completion of the addition, the mixture was refluxed for 4 hours, and then cooled to room temperature to form a precipitate. The precipitate was removed by filtration, and then the remaining reaction mixture was washed with a 5 % potassium hydroxide aqueous solution and further was washed with water. Then, the solvent was distilled off to give a neopentyl glycol diester which was colored yellowish.

The above-obtained neopentyl diester in an amount of 100 ml and 16.7 g of a 5 % Ru/C catalyst (supplied by N.E. Chem. Cat.) were charged into an autoclave, and stirred at a hydrogen pressure of 5 kg/cm² G at 80°C for 5 hours. The reaction mixture was cooled to room temperature, and the catalyst was removed by filtration, to give a colorless transparent liquid. This neopentyl glycol diester was measured for a kinetic viscosity at 40°C with a Ubbellohde viscometer to show 36 cst.

### Comparative Example 21

A neopentyl glycol diester was obtained in the same manner as in Comparative Example 20 except that the olefin mixture was replaced with an olefin mixture which mainly contained olefins having 8 carbon atoms (IP1013, supplied by Idemitsu Petrochemical Co., Ltd., main components = 2,4,4-trimethyl-1-pentene, 2,3,4-trimethyl-2-pentene, 3,4,4-trimethyl-2-pentene, 2,4-dimethyl-2-hexene, 2,3-dimethyl-1-hexene, 3,5-dimethyl-2-hexene, 2,4,4-trimethyl-2-pentene, 2,3,3-trimethyl-2-pentene and 2,3-dimethyl-2-hexene).

The neopentyl glycol diester was measured for a kinetic viscosity at 40°C with a Ubbellohde viscometer to show 35 cst.

**Table 11**

| | **Hydrolysis** | | | Acid value (mgKOH/g) | |
|---|---|---|---|---|---|
| | Amount of water (wt%) | Temperature (°C) | Time (hour) | Before test | After test |
| Ex. 13 | 5.0 | 200 | 3 | 0.01 | 0.01 |
| Ex. 14 | 3.5 | 195 | 5 | 0.01 | 0.01 |
| Ex. 15 | 0 | - | - | 0.01 | 0.35 |
| Ex. 16 | 5.0 | 180 | 3 | 0.01 | 0.20 |
| Ex. 17 | 5.0 | 180 | 10 | 0.01 | 0.07 |
| Ex. 18 | 2.0 | 200 | 3 | 0.01 | 0.07 |
| Ex. 19 | 2.0 | 200 | 10 | 0.01 | 0.03 |
| Ex. 20 | 0 | - | - | 0.01 | 0.61 |
| Ex. 21 | 0 | - | - | 0.01 | 0.63 |
| Ex. = Example, CEx. = Comparative Example | | | | | |

**Table 12**

| | Phase separation temperature on low-temperature side °C | Phase separation temperature on high-temperature side °C | Kinetic viscosity (40°C) cst |
|---|---|---|---|
| Ex. 13 | -72 | 70< | 63 |
| Ex. 14 | -71 | 70< | 63 |
| Ex. 15 | -71 | 70< | 65 |
| Ex. 16 | -71 | 70< | 64 |
| Ex. 17 | -71 | 70< | 64 |
| Ex. 18 | -72 | 70< | 63 |
| Ex. 19 | -72 | 70< | 63 |
| Ex. 20 | -68 | 70< | 36 |
| Ex. 21 | -69 | 70< | 35 |
| Ex. = Example, CEx. = Comparative Example | | | |

## Claims

1. A polyol ester-based lubricant containing, as a base oil, at least one neopentyl polyol ester of formula (I) 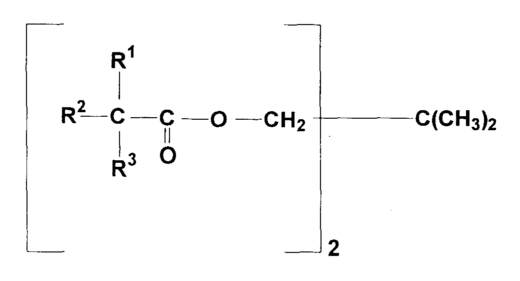 wherein R¹ is an alkyl group having at least 4 carbon atoms and at least one branch and each of R²and R³ is independently an alkyl group having 1 to 13 carbon atoms, provided that the total number of carbon atoms of R¹, R² and R³ is not more than 23, the fatty acid residues of formula (III) 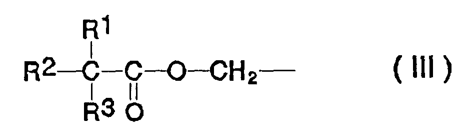 being the same or different, provided that at least 60 % by weight of said at least one polyol ester contains two fatty acid residues of formula (III) containing different numbers of carbon atoms.

2. A lubricant according to claim 1 wherein the low-temperature side phase separation temperature of the said polyol ester is -70°C or lower when the polyol ester is mixed with 1,1,1,2-tetrafluoroethane in a weight ratio of 1:9

3. A lubricant according to claim 1 or 2 wherein the said polyol ester has a saturation moisture absorption of 1,000ppm or less after standing in a closed container at 30°C and a relative humidity of 60% for 30 hours or more.

4. A lubricant according to claim 3 wherein the saturation moisture absorption is 900ppm or less.

5. A lubricant according to any one of the preceding claims wherein the said polyol ester has a pour point of -35°C or less and a volume electric resistivity of at least 1.0 to 10¹⁵ Ω.cm.

6. A lubricant according to any one of the preceding claims wherein the said polyol ester has an acid value of 0.02 mgKOH/g or lower after 10g of the polyol ester having an adjusted water content of 1,000ppm or lower and an acid value of 0.02 mgKOH/g or lower is sealed in a tube with iron wire, copper wire and aluminium wire, each of which has a diameter of 0.15mm and a length of 8cm, and 2g of 1,1,1,2-tetrafluoroethane and allowed to stand at 200°C for 28 days.

7. A lubricant according to any one of the preceding claims further comprising at least one of an antioxidant, a wear preventer and an epoxy compound.

8. A neopentyl polyol ester of formula (I) as defined in any one of claims 1 to 6.

9. A process for the production of a neopentyl polyol ester according to claim 8, which process comprises reacting neopentyl glycol with at least one fatty acid fluoride of formula (II) 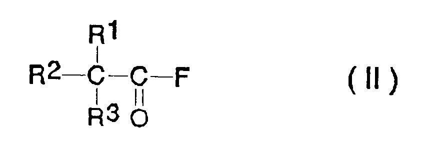 wherein R¹, R² and R³ are each as defined in claim 1,
the or each fatty acid fluoride being obtainable by reacting carbon monoxide, hydrogen fluoride and a monolefin having 4 to 12 carbon atoms wherein at least one of the carbon atoms of the olefinic double bond is a tertiary carbon atom.

10. A process according to claim 9 wherein the or each fatty acid fluoride is obtainable in the presence of 2 to 5 mol of hydrogen fluoride per mole of the monoolefin.

11. A process according to claim 9 or 10 wherein the resulting product is distilled.

12. A process according to claim 11 wherein the distilled product is further purified by hydrolysis-treatment.

13. A process according to claim 12 wherein the hydrolysis-treatment comprises adding at least 3% by weight, based on the reaction mixture, of water and heating to at least 190°C.

## Patentansprüche

1. Schmiermittel auf der Basis von Polyol-ester, umfassend als ein Grundöl mindestens einen Neopentyl-polyol-ester der Formel (I) 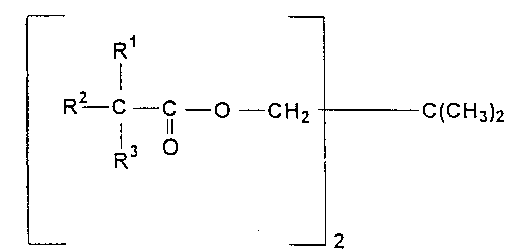 wobei R¹ eine Alkylgruppe mit mindestens 4 Kohlenstoffatomen und mindestens einer Verzweigung ist und R² und R³ jeweils unabhängig eine Alkylgruppe mit 1 bis 13 Kohlenstoffatomen ist, mit der Maßgabe, daß die Gesamtzahl der Kohlenstoffatome von R¹, R² und R³ nicht mehr als 23 beträgt, die Fettsäure-Reste der Formel (III) 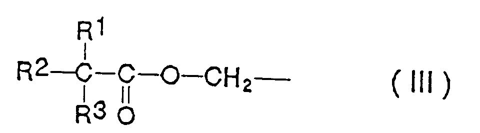 gleich oder verschieden sind, mit der Maßgabe, daß mindestens 60 Gew.% des mindestens einen Polyol-esters zwei Fettsäure-Reste der Formel (III) mit einer unterschiedlichen Anzahl an Kohlenstoffatomen enthalten.

2. Schmiermittel nach Anspruch 1, wobei die Phasentrennungstemperatur auf der Seite der niedrigen Temperatur des Polyol-esters -70°C oder weniger beträgt, wenn der Polyol-ester mit 1,1,1,2-Tetrafluorethan in einem Gewichtsverhältnis von 1:9 vermischt ist.

3. Schmiermittel nach Anspruch 1 oder 2, wobei Der Polyol-ester eine Sättigungs-Feuchtigkeitsabsorption von 1 000 ppm oder darunter aufweist, nachdem er 30 Stunden oder länger in einem geschlossenen Behälter bei 30°C und einer relativen Feuchte von 60% gestanden hat.

4. Schmiermittel nach Anspruch 3, wobei die Sättigungs-Feuchtigkeitsabsorption 900 ppm oder weniger beträgt.

5. Schmiermittel nach einem der vorangehenden Ansprüche, wobei der Polyol-ester einen Gießpunkt von -35°C oder darunter und einen elektrischen Volumenwiderstand von mindestens 1,0 bis 10¹⁵Ω.cm aufweist.

6. Schmiermittel nach einem der vorangehenden Ansprüche, wobei der Polyol-ester eine Säurezahl von 0,02 mg KOH/g oder darunter aufweist, nachdem 10 g des Polyol-esters mit einem eingestellten Wassergehalt von 1 000 ppm oder darunter und einer Säurezahl von 0,02 mg KOH/g oder darunter in einem Rohr mit Eisendraht, Kupferdraht und Aluminiumdraht mit jeweils einem Durchmesser von 0,15 mm und einer Länge von 8 cm und 2 g 1,1,1,2-Tetrafluorethan eingesiegelt und 28 Tage bei 200°C stehen gelassen worden waren.

7. Schmiermittel nach einem der vorangehenden Ansprüche, umfassend ferner mindestens ein Antioxidans, ein Abrieb verhinderndes Mittel und/oder eine Epoxyverbindung.

8. Neopentyl-polyol-ester der Formel (I), wie in einem der Ansprüche 1 bis 6 definiert.

9. Verfahren zur Herstellung eines Neopentyl-polyol-esters nach Anspruch 8, wobei das Verfahren umfaßt das Umsetzen eines Neopentyl-glykols mit mindestens einem Fettsäurefluorid der Formel (II) 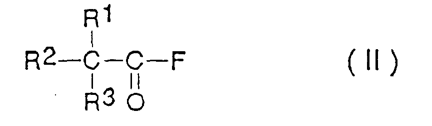 wobei R¹, R² und R³ jeweils wie in Anspruch 1 definiert sind,
wobei das oder jedes Fettsäurefluorid erhältlich ist durch Umsetzen von Kohlenmonoxid, Fluorwasserstoff und einem Monoolefin mit 4 bis 12 Kohlenstoffatomen, wobei mindestens eines der Kohlenstoffatome der olefinischen Doppelbindung ein tertiäres Kohlenstoffatome ist.

10. Verfahren nach Anspruch 9, wobei das oder jedes Fettsäurefluorid erhältlich ist in Gegenwart von 2 bis 5 mol Fluorwasserstoff pro mol Monoolefin.

11. Verfahren nach Anspruch 9 oder 10, wobei das erhaltene Produkt destilliert wird.

12. Verfahren nach Anspruch 11, wobei das destillierte Produkt weiter gereinigt wird durch Hydrolyse-Behandlung.

13. Verfahren nach Anspruch 12, wobei die Hydrolyse-Behandlung die Zugabe von mindestens 3 Gew.% Wasser, bezogen auf das Reaktionsgemisch, und Erhitzen auf mindestens 190°C umfaßt.

## Revendications

1. Lubrifiant à base d'un ester de polyol contenant, comme huile de base, au moins un ester de néopentylglycol de formule (I) : 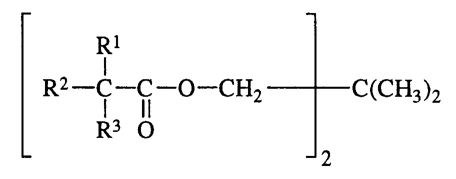 dans laquelle R¹ représente un groupe alkyle comportant au moins 4 atomes de carbone et au moins une ramification, et chacun des R² et R³ représente indépendamment un groupe alkyle comportant de 1 à 13 atomes de carbone, à condition que le nombre total d'atomes de carbone de R¹, R² et R³ ne soit pas supérieur à 23, les résidus d'acide gras de formule (III) : 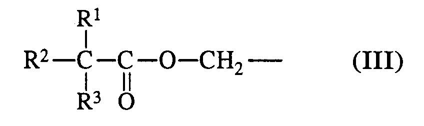 étant identiques ou différents, à condition qu'au moins 60 % en poids dudit ester de polyol, au nombre d'au moins un, contienne deux résidus d'acide gras de formule (III) contenant des nombres d'atome de carbone différents.

2. Lubrifiant selon la revendication 1, dans lequel la température de séparation de phases, dans le cas de faibles températures, dudit ester de polyol vaut -70 °C ou moins quand l'ester de polyol est mélangé avec le 1,1,1,2-tétrafluoroéthane en un rapport pondéral de 1:9.

3. Lubrifiant selon la revendication 1 ou 2, dans lequel ledit ester de polyol présente une quantité d'humidité absorbée à saturation de 1 000 ppm ou moins, après avoir été laissé dans un récipient fermé à 30 °C et sous une humidité relative de 60 % pendant 30 heures ou plus.

4. Lubrifiant selon la revendication 3, dans lequel la quantité d'humidité absorbée à saturation jusqu'à vaut 900 ppm ou moins.

5. Lubrifiant selon l'une quelconque des précédentes revendications, dans lequel ledit ester de polyol présente un point d'écoulement de -35 °C ou moins, et une résistivité électrique volumique d'au moins 1,0 jusqu'à 10¹⁵ Ω.cm.

6. Lubrifiant selon l'une quelconque des précédentes revendications, dans lequel ledit ester de polyol présente un indice d'acide de 0,02 mgKOH/g ou moins après que l'on ait placé dans un tube scellé 10 g d'ester de polyol présentant une teneur en eau ajustée de 1 000 ppm ou moins et un indice d'acide de 0,02 mgKOH/g ou moins, avec un fil de fer, un fil de cuivre et un fil d'aluminium, chacun d'entre eux présentant un diamètre de 0,15 mm et une longueur de 8 cm, et 2 g de 1,1,1,2-tétrafluoroéthane, et qu'on ait laissé reposer à 200 °C pendant 28 jours.

7. Lubrifiant selon l'une quelconque des précédentes revendications, comprenant en outre au moins un des anti-oxydant, agent anti-usure et composé époxydique.

8. Ester de néopentylglycol de formule (I) tel que défini dans l'une quelconque des revendications 1 à 6.

9. Procédé pour produire un ester de néopentylglycol selon la revendication 8, lequel procédé comprend la réaction de néopentylglycol avec au moins un fluorure d'acide gras de formule (II) : 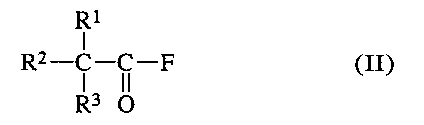 dans laquelle R¹, R² et R³ sont tels définis chacun dans la revendication 1, le fluorure d'acide gras ou chaque fluorure d'acide gras pouvant être obtenu par réaction de monoxyde de carbone, de fluorure d'hydrogène et d'une mono-oléfine comportant de 4 à 12 atomes de carbone, au moins l'un des atomes de carbone de la double liaison oléfinique étant un atome de carbone tertiaire.

10. Procédé selon la revendication 9, dans lequel le fluorure d'acide gras ou chaque fluorure d'acide gras peut être obtenu en présence de 2 à 5 moles de fluorure d'hydrogène par mole de mono-oléfine.

11. Procédé selon la revendication 9 ou 10, dans lequel on distille le produit résultant.

12. Procédé selon la revendication 11, dans lequel le produit distillé est en outre purifié par un traitement d'hydrolyse.

13. Procédé selon la revendication 12, dans lequel le traitement d'hydrolyse comprend l'addition d'au moins 3 % en poids d'eau, par rapport au mélange réactionnel, et le chauffage à une température d'au moins 190 °C.
